(19) Europäisches Patentamt
European Patent Office
Office européen des brevets

(11) Publication number: **0 380 554 B1**

(12) # EUROPEAN PATENT SPECIFICATION

(45) Date of publication of patent specification: **07.07.93**

(51) Int. Cl.5: **C07K 7/16**, A61K 37/34

(21) Application number: **88908781.3**

(22) Date of filing: **04.10.88**

(86) International application number:
**PCT/SE88/00509**

(87) International publication number:
**WO 89/03393 (20.04.89 89/09)**

(54) **VASOACTIVE PEPTIDES.**

(30) Priority: **07.10.87 SE 8703855**

(43) Date of publication of application:
**08.08.90 Bulletin 90/32**

(45) Publication of the grant of the patent:
**07.07.93 Bulletin 93/27**

(84) Designated Contracting States:
**AT BE CH DE FR GB IT LI LU NL SE**

(56) References cited:
**US-A- 3 352 843**
**US-A- 3 558 590**

**Endocrinology 1983, Vol. 112, No. 1, 269-276. Anovulatory Effect of Synthetic Analogs of Arginine Vasotocin in the Rat. Dean W. Cheesman et al.**

**Br. J. Pharmac. (1979), 67, 575-585, Hydroxy Analogues of oxytocin and lysinevasopressin, G.W. Bisset et al.**

**Chemical Abstracts, Vol. 108 (1988), abstract No. 198503f, Eur. J. Pharmacol. 1988, 148 (1), 93-99 (Eng).**

(73) Proprietor: **Ferring AB**
**Soldattorpsvägen 5 Box 30561**
**S-200 62 Malmö(SE)**

(72) Inventor: **MELIN, Per**
**Sveagatan 100 C**
**S-216 15 Malmö(SE)**
Inventor: **TROJNAR, Jerzy**
**Stenöregatan 36**
**S-230 44 Vintrie(SE)**

(74) Representative: **Nilsson, Brita Linnea et al**
**OSCAR GRAHN PATENTBYRA AB, Box 19540**
**S-104 32 Stockholm (SE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

The present invention relates to new vasotocin derivatives, more specifically such vasotocin derivatives as differ from the native hormone in that the vasotocin (VT) structure has been modified at positions 1, 2 and 8.

The new VT derivatives are vasoactive, more particularly by specifically raising the blood pressure, and in some cases have a considerably prolonged effect.

### Background

The peptide hormone vasopressin, produced by the posterior lobe of the pituitary, mainly has two functions, that is the hormone has both an antidiuretic effect (reduced excretion of urine) and a contracting effect on smooth muscles in the vascular wall, the latter effect causing a blood pressure increase and a reduced tendency to bleeding. In clinical use, vasopressin thus has a non-specific effect of short duration.

Today, there is on the market a vasopressin analog having a prolonged effect, namely lysine-vasopressin extended in the N-terminal by three amino acid residues. This vasopressin analog acts as a so-called prohormone or hormonogen, i.e it increases the duration of the vasopressin effect. The extended vasopressin analog has in itself a very small pharmacological effect which does not occur until the extra N-terminal amino acid residues are cleaved by enzymatic hydrolysis and free lysine-vasopressin is formed. Besides the prolonged effect, such a prohormone is advantageous in that the risk of overdosage is minimized by the limited enzyme capacity of the organism determining the plasma levels of the liberated vasopressin. In this manner, it is possible to avoid excessively high plasma levels of vasopressin leading to increased blood pressure which may harm the patient. The above-mentioned vasopressin analog however suffers from major drawbacks by having low potency and, like vasopressin, being non-specific.

There is a need for vasoconstrictive substances for use as bleeding inhibitors and in so-called orthostatic hypotension, i.e. conditions of blood pressure drop following changes of body position. These agents should specifically increase blood pressure, thus having a low antidiuretic effect in order to avoid water intoxication in patients subjected to long-term treatment. Also, it is advantageous if they exhibit an effect of long duration.

### Description of the invention

The present new vasoactive peptides specifically increase blood pressure, i.e. they are pressor-specific, meaning a high ratio of blood pressure- to antidiuretic activity. Furthermore they have a considerably prolonged effect in some cases. The compounds according to the invention are intended to be used in a pharmaceutical composition for inhibiting bleeding and in conditions of blood pressure drop following changes of body position, so-called orthostatic hypotension, and also as general blood pressure increasing agents.

The VT derivatives according to the invention are of the formula

```
      1   2   3   4   5   6   7  8  9
Q1-Hmp-Phe-Ile-Gln-Asn-Cys-Pro-X-Gly-NH2
   |___________________|
```

where
Hmp = a 2-hydroxy-3-mercaptopropionic acid residue

```
(-O-CH-CO-)
    |
    CH2
    |
    S-
```

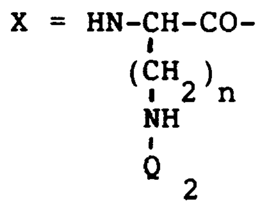

$Q_1$ and $Q_2$ are the same or different and are H or peptide residues made up of the same or different 1 to 3 natural or unnatural L- or D-amino acids, and n is 1, 2 or 3.

The VT derivatives according to the invention can be presented in the form of pharmaceutical compositions in which at least one VT derivative according to the invention is included as active constituent in combination with pharmaceutically acceptable additives and/or diluents. The pharmaceutical compositions according to the invention preferably are in the form of preparations suitable for parenteral administration. They are suitably administered by injection, infusion or intranasal application. The diluent may be e.g. a physiological saline solution.

A pharmaceutical composition according to the invention may contain a specifically blood pressure increasing derivative having a relatively short duration for providing an instant effect, in combination with a specifically blood pressure increasing derivative having a long duration for providing a prolongation of the effect.

Preparation of the VT derivatives according to the invention

The VT derivatives according to the invention can be prepared by methods analogous with those which are known in the peptide field.

For instance, the compounds according to the invention can be prepared in conventional manner by coupling amino acids stepwise to one another in liquid phase, e.g. as disclosed by Law, H.B. & Du Vigneaud, V. in Journal of the American Chemical Society 82, (1960) 4579-4581, Zhuze, A.L., Jŏst, K., Kasafirek, E. & Rudinger, J. in Collection of Czechoslovak Chemical Communications 29, (1964), 2648-2662, and modified by Larsson, L.-E., Lindeberg, G., Melin, P. / Pliška, V. in Journal of Medicinal Chemistry 21, (1978), 352-356. The coupling of the amino acids to one another, yielding so-called peptide bonds, can also be effected with a solid phase (generally a resin) as starting material to which the C-terminal of the first amino acid is coupled, whereupon the C-terminal of the next amino acid is coupled to the N-terminal of the first amino acid and so on. Finally, the finished peptide is liberated from the solid phase. In the Examples hereinbelow, this so-called solid phase technique has been used in accordance with the disclosure of Merrifield, R.B., J. Am. Chem. Soc. (1963) 85, 2149, Merrifield, R.B. Biochemistry (1964), 3, 1385 and König, W., Geiger, R., Chem. Ber. (1970), 103, 788.

General description of synthesis

All the VT derivatives prepared in the Examples given below were synthesised on an Applied Biosystems 430A Peptide Synthesizer using a double coupling program with a termination step after the second coupling. The resin used was of 4-methylbenzhydrylamine type with a theoretical loading of 0.66 meq/g (Peptides International, Louisville, KY, USA). The final product of the synthesis was dried in vacuo overnight. The peptide was then cleaved from the resin by treatment with liquid hydrogen fluoride in the presence of anisole and ethyl-methyl-sulphide as scavengers (HF:anisole:EMS - 10:05:05). After removal of hydrogen fluoride by evaporation, the resin was suspended in ethyl acetate (100 ml) and filtered. The solid was washed on filter with additional ethyl acetate (3x100 ml), and the cleaved peptide was extracted with acetic acid (100 ml). The extract was promptly diluted to a volume of 2 l with 20% acetic acid in methanol and treated with 0.1 M solution of iodine in methanol until a faint brown colour remained. Then a Dowex 1x8 ion exchanger in acetate form (3 g) (Bio-Rad, Richmond, CA) was added and the mixture filtered. The filtrate was evaporated and the residue freeze-dried from 1% acetic acid in water. The product was then purified by reversed phase liquid chromatography on a column filled with Vydac 20-25 $\mu$ (Separation Group, CA) in a suitable system containing acetonitrile in 0.1% trifluoroacetic acid water solution. The samples collected from the column were analysed by analytical high performance liquid chromatography (HPLC) (Varian 5500, Sunnyvale, CA) equipped with a Bondapak C18 column (Millipore, Milford, Mass.). Fractions containing pure substance were pooled, the solvent was evaporated and the product freeze-dried from 1% acetic acid in water. The final HPLC analysis was performed on ready product, and the structure of the

peptide was confirmed by amino acid analysis and fast atom bombardment mass spectrometry (FAB MS).

All amino acids used during the synthesis were L-amino acids, and they were protected with a tert-butoxy-carbonyl group at the α-amino function. The side chains were protected as follows:
Ser(BZL), Thr(BZL), Tyr(2-BrCbz), Lys(2-ClCbz), Orn(Cbz), Asp(BZL), Glu(BZL), Arg(Tos), Cys(Mob), Hmp-(Acm), Cys(Acm), Orm(Boc).

The abbreviations within brackets are:

| | |
|---|---|
| BZL = | benzyl; |
| 2-BrCbz = | 2-bromocarbobenzoxy; |
| 2-ClCbz = | 2-chlorocarbobenzoxy; |
| Cbz = | carbobenzoxy; |
| Tos = | tosyl; |
| Mob = | 4-methoxybenzyl; |
| Acm = | acetamidomethyl; and |
| Boc = | t-butyloxycarbonyl |

The amino acid derivatives were supplied by Bachem AG, Switzerland.

## EXAMPLE 1

1-Hmp-2-Phe-8-Orn-VT
[$Q_1$ = H, n = 3 and $Q_2$ = H]

The peptide was synthesised according to the general description. 2-hydroxy-mercaptopropionic acid [S-(p-methoxy)benzyl] was used for position 1. Purity (HPLC): 99.5% (27% acetonitrile in 0.1% TFA, retention time 8.13 min at 2 ml/min, detection at 223 nm).

Amino acid analysis:

Asp 1.05; Glu 1.02; Pro 1.00; Gly 0.94; Ile 1.00; Phe 0.95; Orn 0.91.

## EXAMPLE 2

1-Hmp-2-Phe-8-Dab-VT
[$Q_1$ = H, n = 2, $Q_2$ = H]

This analog was synthesised according to the general description and Example 1. Boc Dab(Cbz) was used for coupling at position 8 (Dab = 2,4-diaminobutyric acid).
Purity (HPLC): 99.5% (24% acetonitrile/0.1% TFA,
retention time 6.0 min at 2 ml/min,
detection at 223 nm).

Amino acid analysis:

Asp 1.00; Glu 1.03; Pro 0.97; Gly 1.08; Ile 0.98; Phe 0.99.

## Example 3

1-Hmp-2-Phe-8-Orn(Ala)-VT
[$Q_1$ = H, n = 3, $Q_2$ = alanyl]

The synthesis of this compound was performed as in Example 1 with the exception of the amino acid at position 8. Boc-Orn(Ala)-OH was used instead of Boc-Orn(Cbz)OH.
Purity (HPLC): 97.8% (27% acetonitrile/0.1% TFA;
retention time 10.09 min at 2 ml/min,
detection at 223 nm).

Amino acid analysis:

Asp 1.05; Glu 0.95; Pro 1.03; Gly 1.0; Ala 0.98; Cys 0.87; Ile 0.73; Phe 0.90; Orn 0.96.

EXAMPLE 4

$Gly^o$-1-Hmp-2-Phe-8-Orn-VT
[$Q_1$ Gly, n = 3 and $Q_2$ = H]

The protected nonapeptide Hmp(Acm)-Phe-Ile-Gln-Asn-Cys(Acm)-Pro-Orn(Boc)-Gly-$NH_2$ (500 mg; prepared by solid phase method according to the general description) was dissolved in DMF (25 ml). Pyridine (5 ml) and previously formed Boc Gly anhydride (10 equivalents) were added at 0°C under vigorous stirring. The reaction mixture was left overnight at room temperature. The product was isolated by precipitation with ethanol and diethyl ether, filtration and drying in vacuo.

The protected decapeptide (Boc-Gly-Hmp(Acm)-Phe-Ile-Glu-Asn-Cys(Acm)-Pro-Orn(Boc)-Gly-$NH_2$ (342 mg, 0.25 mmole) was dissolved in 70% methanol (1500 ml) and a solution of iodine (160 mg) in methanol (375 ml) was added dropwise under stirring. Dowex 1x8 (50 g acetate form) was added and the mixture filtered. The filtrate was evaporated and the residue dried by evaporation with toluene followed by drying in dessicator in vacuo.

The product was then treated with TFA (10 ml) containing anisole (1 ml), stirred for 15 min and then treated with diethyl ether (100 ml). The precipitation was separated by filtration and dried in vacuo.

The product was purified by reversed phase liquid chromatography.

Purity (HPLC): 95% (24% acetonitrole/0.1% TFA,
retention time 5.8 min at 1 ml/min,
detection at 223 nm).

The structure was confirmed by amino acid analysis and FAB MS analysis.

Amino acid analysis:

Asp 1.29; Glu 1.00; Pro 0.96; Gly 2.35; Cys 0.44; Ile 0.82; Phe 0.97; Orn 1.12.

Pharmacological tests

Vasotocin derivatives according to the invention have been tested for potency of both blood pressure and antidiuretic activity in a so-called 4-point test, i.e. the activity of the test substances has been related to a standard preparation (AVP = arginine-vasopressin), and the effects of two dose levels for each substance have been analysed. In addition, two known pressor-specific analogs (VT derivatives) have been tested for a comparison, namely 1-mpa-2-Phe-8-Orn-VT (mpa = 3-mercaptopropionyl) (Huguenin, R.L., Boissonnas, R.A., 1966. Helv. Chim. Acta, 49, 711), and 2-Phe-8-Orn-VT (SE-332993, Sandoz).

Blood pressure tests were carried out on anaesthetised Sprague Dawley rats (about 250 g), previously treated with dibenamine (Dekanski, J., 1952. Br. J. Pharmacol. 7, 567). Maximal blood pressure increase after intravenous injections of peptide was used as a measure of the effect, expressed as intensity.

In addition to potency determination based on effect intensity, a measure of the length of the effect has been stated (index of persistence (I.P.), Pliška, V., 1966. Arzheim. Forsch. 16, 886). This dimensionless factor is a measure of the effect duration of the respective analog in relation to the standard AVP.

Antidiuretic potency was determined with the aid of anaesthetised water-loaded Sprague Dawley rats (200 g) (Larsson, L.E., Lindeberg, G., Melin, P. and Pliška, V., 1978, J. Med. Chem. 21, 353). Maximal increase of urine conductivity after intravenous injections was used as effect parameter.

In these two tests, a comparison was made between the effects of the respective derivative and the effect of a standard preparation, AVP, and potency was determined with the aid of a 4-point test and is indicated in international units per milligram (IU/mg) (Stürmer, E., i: Handbook of Experimental Pharmacology, 1966, Vol 23, pp 130-189).

The specificity in respect of blood pressure is indicated by the ratio of potency blood pressure/potency antidiuresis (BP/AD).

The pharmacological results are given in Table 1.

From Table 1 appears that compounds 4 and 5 according to the invention exhibit a very high potency in respect of blood pressure increase in relation to the known derivatives 2 and 3, and an equivalent or higher specificity than the known compounds 1, 2 and 3. The very high potency in respect of blood pressure increase makes it possible to administer compounds 4 and 5 in considerably lower doses than the known pressor-specific substances 2 and 3. It further appears from Table 1 that compounds 6 and 7, which can be regarded as prodrugs, have surprisingly high blood pressure potency and adequate specificity. Furthermore, they exhibit a considerably prolonged effect as compared with the known substances 1, 2 and 3.

Example of the preparation of a pharmaceutical composition

The VT derivative is dissolved in distilled water together with mannitol. The solution is poured into an ampoule, subjected to freeze-drying and is sealed. The contents in the ampoule can then, when desired, be diluted with an isotonic saline solution to a concentration suitable for administration.

TABLE 1

| ANALOG | BLOOD PRESSURE BP IU/mg | BLOOD PRESSURE BP I.P (measure of duration of effect | ANTIDIURESIS AD IU/mg | BP/AD (measure of specificity) |
|---|---|---|---|---|
| 1. AVP (known) | 408 | 1.0 | 408 | 1.0 |
| 2. 2-Phe-8-Orn-VT (known) | 94 ± 15 | 2.2 ± 0.9 | 2.6 | 36 |
| 3. 1-mpa-2-Phe-8-Orn-VT (known) | 66 ± 12 | 1.7 + 0.4 | 4.4 ± 0.3 | 15 |
| 4. 1-Hmp-2-Phe-8-Orn-VT (Ex 1) | 339 ± 13 | 3.1 ± 0.5 | 4.9 ± 0.4 | 69 |
| 5. 1-Hmp-2-Phe-8-Dab-VT (Ex 2) | 657 ± 32 | 3.6 ± 0.9 | 18.2 ± 2.1 | 36 |
| 6. 1-Hmp-2-Phe-8-Orn(Ala)-VT (Ex 3) | 48 ± 2.2 | 9.1 ± 2.0 | 3.9 ± 0.5 | 12 |
| 7. $Gly^{O}$-1-Hmp-2-Phe-8-Orn-VT (Ex 4) | 69 ± 6.5 | 15.2 ± 3.1 | 2.8 ± 0.39 | 25 |

## Claims

1. Vasotocin derivative of the formula

$$\begin{array}{l} \quad\;\; 1 \quad\;\; 2 \quad\;\; 3 \quad\;\; 4 \quad\;\; 5 \quad\;\; 6 \quad\; 7 \;\; 8 \;\; 9 \\ Q_1\text{-Hmp-Phe-Ile-Gln-Asn-Cys-Pro-X-Gly-}NH_2 \end{array}$$

where
Hmp is a 2-hydroxy-3-mercaptopropionic acid residue

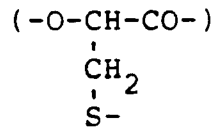

X is 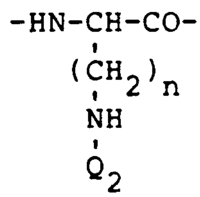

$Q_1$ and $Q_2$ are the same or different and are H or peptide residues made up of the same or different 1 to 3 natural or unnatural L- or D-amino acids, and n is 1, 2 or 3.

2. Vasotocin derivative as claimed in claim 1, **characterised** in that
$Q_1$ is H,
n is 3, and $Q_2$ is H.

3. Vasocotin derivative as claimed in claim 1, **characterised** in that
$Q_1$ is H,
n is 2, and $Q_2$ is H.

4. Vasotocin derivative as claimed in claim 1, **characterised** in that
$Q_1$ is H,
n is 3, and $Q_2$ is alanyl.

5. Vasotocin derivative as claimed in claim 1, **characterised** in that
$Q_1$ is glycyl
n is 3, and $Q_2$ is H.

6. Pharmaceutical composition, **characterised** in that it comprises at least one vasotocin derivative as claimed in claim 1 as active constituent in combination with pharmaceutically acceptable additives and/or diluents.

7. Pharmaceutical composition as claimed in claim 6, **characterised** in that it is in the form of a preparation suitable for parenteral administration.

8. Pharmaceutical composition as claimed in claim 7, **characterised** in that it is in form of a solution suitable for intranasal administration.

**Patentansprüche**

**1.** Vasotocinderivat der Formel

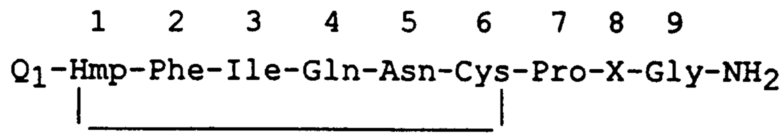

wobei
HmP = ein 2-Hydroxy-3-mercaptopropionsäurerest

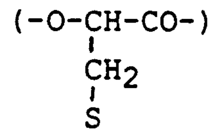

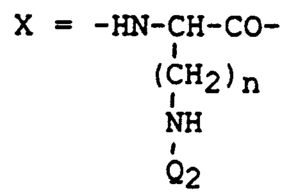

$Q_1$ und $Q_2$ sind gleich oder unterschiedlich und bedeuten H oder Peptidreste, die aus 1 bis 3 gleichen oder unterschiedlichen natürlichen oder unnatürlichen L- oder D-Aminosäuren hergestellt sind, und
n ist 1, 2 oder 3.

**2.** Vasotocinderivat nach Anspruch 1, dadurch **gekennzeichnet,** daß $Q_1$ H ist, n 3 ist und $Q_2$ H ist.

**3.** Vasotocinderivat nach Anspruch 1, dadurch **gekennzeichnet,** daß $Q_1$ H ist, n 2 ist und $Q_2$ H ist.

**4.** Vasotocinderivat nach Anspruch 1, dadurch **gekennzeichnet,** daß $Q_1$ H ist, n 3 ist und $Q_2$ Alanyl ist.

**5.** Vasotocinderivat nach Anspruch 1, dadurch **gekennzeichnet,** daß $Q_1$ Glycyl ist, n 3 ist, und $Q_2$ H ist.

**6.** Pharmazeutische Zusammensetzung, dadurch **gekennzeichnet,** daß sie mindestens ein Vasotocinderivat nach Anspruch 1 als aktiven Bestandteil in Kombination mit pharmazeutisch geeigneten Additiven und/oder Verdünnungsmitteln enthält.

**7.** Pharmazeutische Zusammensetzung nach Anspruch 6, dadurch **gekennzeichnet,** daß sie in Form einer Zubereitung, die sich für parenterale Verabreichung eignet, vorliegt.

**8.** Pharmazeutische Zusammensetzung nach Anspruch 7, dadurch **gekennzeichnet,** daß sie in Form einer für intranasale Verabreichung geeigneten Lösung vorliegt.

## Revendications

1. Dérivé de vasotocine de formule

$$\overset{1}{Q_1}\text{-}\overset{2}{\text{Hmp}}\text{-}\overset{3}{\text{Phe}}\text{-}\overset{4}{\text{Ile}}\text{-}\overset{5}{\text{Gln}}\text{-}\overset{6}{\text{Asn}}\text{-}\overset{7}{\text{Cys}}\text{-}\overset{8}{\text{Pro}}\text{-}\overset{9}{\text{X}}\text{-Gly-}NH_2$$

dans laquelle
Hmp est un résidu d'acide 2-hydroxy-3-mercaptopropionique

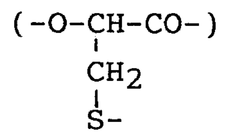

X est

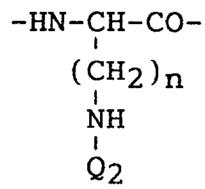

$Q_1$ et $Q_2$ sont identiques ou différents et sont H ou des résidus peptidiques constitués de 1 à 3 L- ou D-aminoacides naturels ou non, identiques ou différents, et
n est 1, 2 ou 3.

2. Dérivé de vasotocine selon la revendication 1, caractérisé en ce que $Q_1$ est H, n est 3 et $Q_2$ est H.

3. Dérivé de vasotocine selon la revendication 1, caractérisé en ce que $Q_1$ est H, n est 2 et $Q_2$ est H.

4. Dérivé de vasotocine selon la revendication 1, caractérisé en ce que $Q_1$ est H, n est 3 et $Q_2$ est alanyle.

5. Dérivé de vasotocine selon la revendication 1, caractérisé en ce que $Q_1$ est glycyle, n est 3 et $Q_2$ est H.

6. Composition pharmaceutique caractérisée en ce qu'elle comprend au moins un dérivé de vasotocine selon la revendication 1 en tant que constituant actif, en combinaison avec des additifs et/ou des diluants pharmaceutiquement acceptables.

7. Composition pharmaceutique selon la revendication 6, caractérisée en ce qu'elle est sous forme d'une préparation convenant à l'administration parentérale.

8. Composition pharmaceutique selon la revendication 7, caractérisée en ce qu'elle est sous forme d'une solution convenant à l'administration intranasale.